# EUROPÄISCHE PATENTSCHRIFT

(11) **EP 0 529 426 B1**
(45) Veröffentlichungstag und Bekanntmachung des Hinweises auf die Patenterteilung: **05.07.1995**
(21) Anmeldenummer: 92113803.8
(22) Anmeldetag: 13.08.1992
(51) Int. Cl.: C07C 201/12, C07C 227/04, C07C 205/58, C07C 205/57, C07C 229/56

(54) **Verfahren zur Herstellung von Nitrobenzoesäure und Anthranilsäuren**
Process for the preparation of nitrobenzoic and anthranilic acids
Procédé de préparation des acides nitrobenzoiques et anthraniliques

(30) Priorität: 27.08.1991 DE 4128351
(43) Veröffentlichungstag der Anmeldung: 03.03.1993
(73) Patentinhaber: BASF Aktiengesellschaft, 67063 Ludwigshafen (DE)
(72) Erfinder: Hagen, Helmut, Dr., W-6710 Frankenthal (DE); Dupuis, Jacques, Dr., W-6700 Ludwigshafen (DE)

(56) Entgegenhaltungen:
- EP-A- 0 282 778
- EP-A- 0 460 544
- DE-A- 3 409 244
- CHEMISTRY AND INDUSTRY. CHEMISTRY AND INDUSTRY REVIEW Nr. 41, 8. Oktober 1966,LETCHWORTH GB Seiten 1722 - 1723 P. S. RADHAKRISHNA MURTI, S. C. PATI'Oxidation of Toluene and Some of Its Derivatives by V(V)'

## Beschreibung

Die vorliegende Erfindung betrifft ein verbessertes Verfahren zur Herstellung von Nitrobenzoesäuren der allgemeinen Formel I 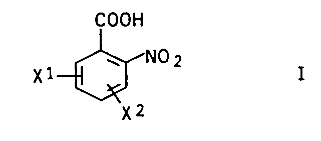
in der X¹ und X² Fluor, Chlor, Brom, Wasserstoff oder Nitro bedeuten, durch Oxidation des entsprechenden Nitrotoluols der allgemeinen Formel II 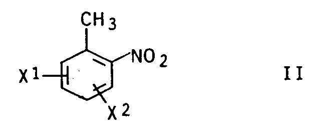
mit Salpetersäure
sowie die Herstellung von Anthranilsäuren der allgemeinen Formel III 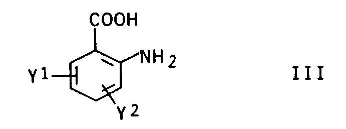
in der Y¹ und Y² Fluor, Chlor, Brom, Wasserstoff oder Amino bedeuten, durch Reduktion der erfindungsgemäß erhaltenen Nitrobenzoesäuren I.

Nitrobenzoesäuren der Formel I sind wertvolle Zwischenprodukte für die Herstellung von Anthranilsäuren der Formel III, die wiederum wichtige Ausgangsverbindungen für die Herstellung von Farbstoffen darstellen.

Die Nitrobenzoesäuren I sind bekanntermaßen durch Oxidation des entsprechenden Nitrotoluols II zugänglich. Bei dem in der DE-A-34 09 244 beschriebenen Verfahren erfolgt diese Oxidation mit verdünnter Salpetersäure unter hohem Druck und bei hoher Temperatur, was die Verwendung von kostspieligen druckfesten Reaktionsapparaturen erforderlich macht.

Die Nitrobenzoesäuren I können nach der DE-A-34 09 244 dann durch katalytische Hydrierung in die entsprechenden Anthranilsäuren III überführt werden.

Der Erfindung lag die Aufgabe zugrunde, Nitrobenzoesäuren I in guten Ausbeuten und guten Reinheiten in verfahrenstechnisch einfacher und wirtschaftlicher Weise zugänglich zu machen und damit auch eine vorteilhafte Herstellung der Anthranilsäuren III zu ermöglichen.

Demgemäß wurde ein Verfahren zur Herstellung von Nitrobenzoesäuren der allgemeinen Formel I 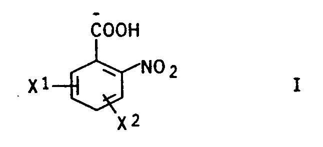
in der X¹ und X² Fluor, Chlor, Brom, Wasserstoff oder Nitro bedeuten, durch Oxidation des entsprechenden Nitrotoluols der allgemeinen Formel II 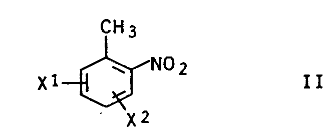
mit Salpetersäure gefunden, welches dadurch gekennzeichnet ist, daß man die Oxidation in Gegenwart von Schwefelsäure und Vanadium(V)verbindungen vornimmt.

Die erfindungsgemäß hergestellten Nitrobenzoesäuren I können dann nach ihrer Isolierung durch Reduktion in Anthranilsäuren der Formel III 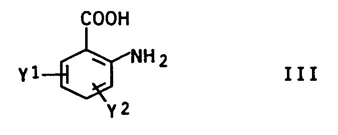
in der Y¹ und Y² Fluor, Chlor, Brom, Wasserstoff oder Amino bedeuten, überführt werden.

Für das erfindungsgemäße Verfahren geeignete Vanadium(V)verbindungen sind vorzugsweise solche, die in Säuren, besonders in Schwefelsäure bzw. in Gemischen von Schwefelsäure und Salpetersäure löslich sind, also neben dem Nitrat und Acetat bevorzugt das Sulfat und besonders bevorzugt das Oxid. Sie können einzeln oder in Form von Gemischen verwendet werden.

In der Regel werden 0,01 bis 0,2, vorzugsweise 0,08 bis 0,12 Moläquivalente Vanadium(V)verbindung pro mol II eingesetzt.

Vorzugsweise wird die Oxidation in Gegenwart von 60 bis 85 gew.-%iger Schwefelsäure als Reaktionsmedium durchgeführt. Die Menge Schwefelsäure ist frei wählbar, es empfiehlt sich jedoch, pro mol II 0,5 bis 3 mol, bevorzugt 1 bis 2 mol Schwefelsäure zu verwenden.

Die Konzentration der für die erfindungsgemäße Oxidation verwendeten Salpetersäure kann stark variiert werden, vorteilhaft sind jedoch Konzentrationen von 40 bis 100 Gew.-%, vorzugsweise 50 bis 70 Gew.-%. Die Menge an Salpetersäure beträgt in der Regel 2 bis 8, bevorzugt 3 bis 5 mol pro mol II.

In der Regel führt man die Reaktion bei 130 bis 190°C, vorzugsweise bei 165 bis 180°C durch.

Von besonderem Vorteil ist, daß die Umsetzung unter Normaldruck vorgenommen werden kann. Eine Reaktionsführung unter Druck ist jedoch nicht ausgeschlossen.

Verfahrenstechnisch geht man zweckmäßigerweise so vor, daß man das Nitrotoluol II und die Vanadium(V)verbindung in der Schwefelsäure suspendiert und nach Erhitzen auf etwa 130 bis 190°C die Salpetersäure zudosiert.

Während der Umsetzung, die im allgemeinen 8 bis 12 Stunden in Anspruch nimmt, kann verdünnte Salpetersäure im Gemisch mit organischer Phase abdestilliert werden. Die organische Phase wird dann zweckmäßigerweise in das Reaktionsgefäß zurückgeführt.

Man kann das erfindungsgemäße Verfahren sowohl diskontinuierlich als auch kontinuierlich, z.B. in einer Rührkesselkaskade, durchführen.

Die Aufarbeitung des Reaktionsgemisches auf die Nitrobenzoesäuren I erfolgt vorzugsweise durch wiederholte Extraktion mit einem säurestabilen organischen Lösungsmittel, wie Nitrobenzol, Mono-, Di- und Trichlorbenzolen, insbesondere o-Dichlorbenzol, bei 90 bis 150°C, vorzugsweise 135 bis 145°C. Die Nitrobenzoesäuren I können durch Abkühlen des erhaltenen Extraktes auf etwa 0 bis 5°C ausgefällt und durch übliche Filtration isoliert werden.

Eine weitere Reinigung der Produkte I ist in der Regel nicht erforderlich. Sie können vielmehr, gegebenenfalls nach Trocknung, direkt für Folgeumsetzungen eingesetzt werden. Die Reinheitsüberprüfung erfolgt in der Regel chromatographisch.

Diese Art der Aufarbeitung hat den Vorteil, daß sowohl die abgetrennte schwefelsaure Phase als auch das Extraktionsmittel wiederholt zur Umsetzung bzw. zur Extraktion verwendet werden können.

Man erhält die Nitrobenzoesäuren I nach dem erfindungsgemäßen Verfahren auf verfahrenstechnisch einfache und damit wirtschaftliche Weise in guter Ausbeute und hoher Reinheit.

Die erfindungsgemäß hergestellten Nitrobenzoesäuren I können vorteilhaft als Ausgangsprodukte für die Herstellung von Anthranilsäuren III dienen.

Die hierfür erforderliche Reduktion kann nach an sich bekannten Methoden, vorzugsweise durch katalytische Hydrierung, erfolgen (vgl. Houben-Weyl, "Methoden der Organischen Chemie", Band XI/1, S. 360-492 (1957)).

Als Katalysatoren kommen dabei vor allem Raney-Nickel und Palladium sowie insbesondere Platin in Betracht. Palladium und Platin werden zweckmäßigerweise in Form eines Trägerkatalysators, vorzugsweise auf Kohle, eingesetzt.

Die katalytische Hydrierung wird im allgemeinen in einem Alkohol, wie Ethanol, Propanol, Isopropanol und insbesondere Methanol, bei Temperaturen von 20 bis 60°C und einem Wasserstoff-Druck von bis zu 2 bar durchgeführt.

Nach beendeter Hydrierung wird zweckmäßigerweise vom Katalysator abfiltriert. Die Anthranilsäuren III können dann durch Einengen des Filtrats oder besonders vorteilhaft durch direkte Sprühtrocknung der Reduktionslösung isoliert werden.

Auch die Anthranilsäuren III fallen in so hoher Reinheit an, daß sie direkt für weitere Zwecke wie die Farbstoffherstellung eingesetzt werden können.

Die Herstellung der Anthranilsäuren III über die erfindungsgemäße Oxidation von Nitrotoluolen II und anschließende Reduktion der so erhaltenen Nitrobenzoesäuren I stellt einen vorteilhaften Syntheseweg dar.

### Beispiele

### Beispiel 1

### Herstellung von 2-Chlor-6-nitrobenzoesäure

Eine Mischung aus 343 g (2 mol) 2-Chlor-6-nitrotoluol, 80 g (0,4 mol) Vanadiumpentoxid, 2609 g 96 gew.-%iger Schwefelsäure und 731 g Wasser wurde auf 175°C erhitzt. Bei dieser Temperatur wurden innerhalb von 10,5 h 610 g (439 ml; 6,3 mol) 65 gew.-%ige Salpetersäure zudosiert.

In dieser Zeit wurden in einer auf etwa 100°C vorgeheizten Destillationsvorlage 347 g Destillat erhalten, von dem die organische Phase (insgesamt 37 g) in den Reaktionskessel zurückgepumpt wurde. Nach beendeter Salpetersäurezugabe wurde die Reaktionsmischung noch 30 min nachgerührt und anschließend auf 140°C abgekühlt.

Zur Isolierung des Zielproduktes wurde einmal mit 1000 ml und zweimal mit 500 ml o-Dichlorbenzol extrahiert. Die vereinigten organischen Phasen (3005 g) wurden in einem Fällkessel innerhalb von etwa 4 h auf 0 bis 5°C abgekühlt. Die auf diese Weise ausgefällte 2-Chlor-6-nitrobenzoesäure wurde abfiltriert und bei 50°C im Vakuum getrocknet.

Es wurden 267 g 2-Chlor-6-nitrobenzoesäure mit einem Gehalt von > 98 % (Ausbeute 65 %) erhalten, die direkt zur Herstellung von 6-Chloranthranilsäure eingesetzt werden konnten.

### Beispiel 2

### Herstellung von 6-Chloranthranilsäure

262 g (1,3 mol) der in Beispiel 1 erhaltenen 2-Chlor-6-nitrobenzoesäure wurden unter Stickstoffbegasung in einem druckfesten Kessel in 1583 g Methanol gelöst. Nach Zugabe von 25 g eines Platinkatalysators (5 Gew.-% Platin auf Aktivkohle) wurde der Kessel verschlossen und nach Erwärmung des Kesselinhalts auf 50°C mit 1 l Wasserstoff gespült. Bei einem Überdruck von etwa 0,5 bar wurden 7,8 g (87 l; 3,9 mol) Wasserstoff aufgepreßt.

Zur Isolierung des Zielproduktes wurde der Katalysator nach beendeter Wasserstoffaufnahme abfiltriert, und das Filtrat wurde sprühgetrocknet.

Es wurden 225 g 6-Chloranthranilsäure mit einem Gehalt von > 98 % erhalten, was einer Ausbeute von 98 % entspricht.

## Patentansprüche

1. Verfahren zur Herstellung von Nitrobenzoesäuren der allgemeinen Formel I 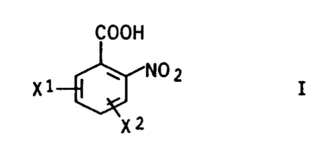 in der X¹ und X² Fluor, Chlor, Brom, Wasserstoff oder Nitro bedeuten, durch Oxidation des entsprechenden Nitrotoluols der allgemeinen Formel II 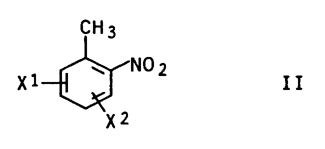 mit Salpetersäure, dadurch gekennzeichnet, daß man die Oxidation in Gegenwart von Schwefelsäure und Vanadium(V)-verbindungen vornimmt.

2. Verfahren zur Herstellung von Anthranilsäuren der allgemeinen Formel III 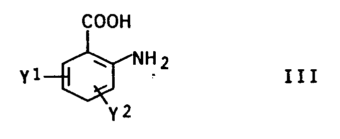 in der Y¹ und Y² Fluor, Chlor, Brom, Wasserstoff oder Amino bedeuten, durch Oxidation des entsprechenden Nitrotoluols der allgemeinen Formel II 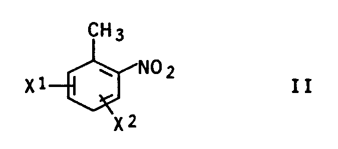 in der X¹ und X² Fluor, Chlor, Brom, Wasserstoff oder Nitro bedeuten, mit Salpetersäure zur Nitrobenzoesäure der allgemeinen Formel I 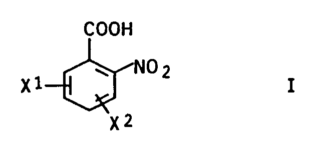 und Isolierung und anschließende Reduktion dieser Nitrobenzoesäure, dadurch gekennzeichnet, daß man die Oxidation in Gegenwart von Schwefelsäure und Vanadium(V)verbindungen vornimmt.

## Claims

1. A process for preparing nitrobenzoic acids of the general formula I 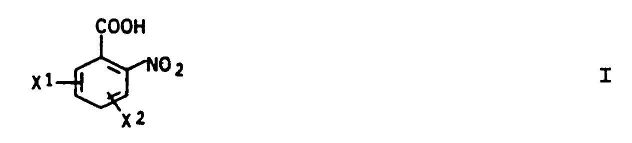 where X¹ and X² are each fluorine, chlorine, bromine, hydrogen or nitro, by oxidizing the corresponding nitrotoluene of the general formula II 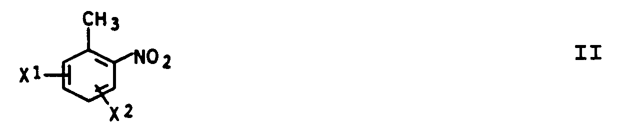 with nitric acid, which comprises carrying out the oxidation in the presence of sulfuric acid and vanadium(V) compounds.

2. A process for preparing anthranilic acids of the general formula III 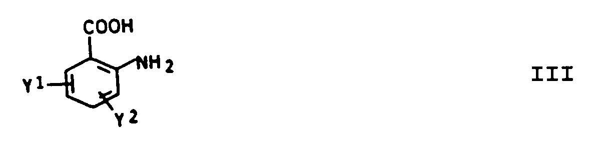 where Y¹ and Y² are each fluorine, chlorine, bromine, hydrogen or amino, by oxidizing the corresponding nitrotoluene of the general formula II 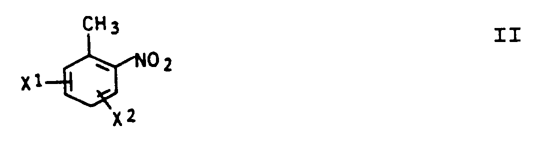 where X¹ and X² are each fluorine, chlorine, bromine, hydrogen or nitro, with nitric acid to obtain nitrobenzoic acid of the general formula I 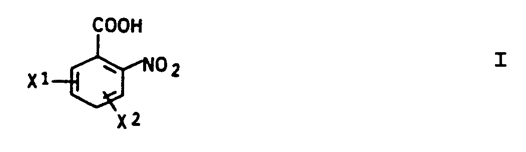 and isolating and subsequently reducing this nitrobenzoic acid, which comprises carrying out the oxidation in the presence of sulfuric acid and vanadium(V) compounds.

## Revendications

1. Procédé de préparation d'acides nitrobenzoïques de formule générale I 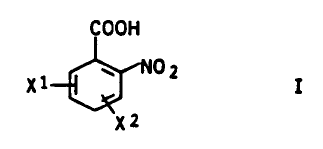 dans laquelle X¹ et X² représentent des atomes de fluor, de chlore, de brome, d'hydrogène ou des groupements nitro, par oxydation du nitrotoluène correspondant de formule générale II 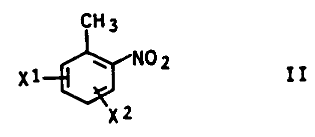 avec de l'acide nitrique, caractérisé en ce que l'on procède à l'oxydation en présence d'acide sulfurique et de composés vanadiques.

2. Procédé de préparation d'acides anthraniliques de formule générale III 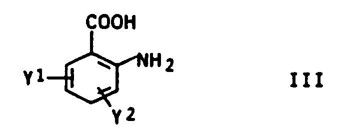 dans laquelle Y¹ et Y² représentent des atomes de fluor, de chlore, de brome, d'hydrogène ou des groupements amino, par oxydation du nitrotoluène correspondant de formule générale II 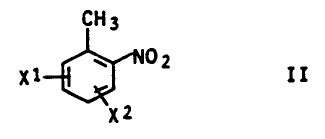 dans laquelle X¹ et X² représentent des atomes de fluor, de chlore, de brome, d'hydrogène ou des groupements nitro, avec de l'acide nitrique pour obtenir l'acide nitrobenzoïque de formule générale I 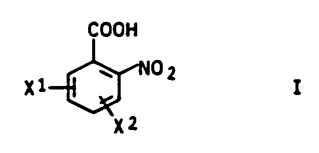 et par isolement, puis reduction de cet acide nitrobenzoïque, caractérisé en ce que l'on procède à l'oxydation en présence d'acide sulfurique et de composés vanadiques.
